# EUROPÄISCHE PATENTSCHRIFT

(11) **EP 1 360 928 B1**
(45) Veröffentlichungstag und Bekanntmachung des Hinweises auf die Patenterteilung: **24.05.2006**
(21) Anmeldenummer: 03003472.2
(22) Anmeldetag: 15.02.2003
(51) Int. Cl.: A61B 1/32

(54) **Spreizlaryngoskop**
Laryngoscope that can be spread
Laryngoscope à section écartable

(30) Priorität: 07.05.2002 DE 10220497
(43) Veröffentlichungstag der Anmeldung: 12.11.2003
(73) Patentinhaber: Richard Wolf GmbH, 75438 Knittlingen (DE)
(72) Erfinder: Dietzel, Daniel, Dipl.-Ing. (FH), 65779 Kelkheim (DE); Heckele, Helmut, 75438 Knittlingen (DE)
(74) Vertreter: Hemmer, Arnd

(56) Entgegenhaltungen:
- WO-A-01/34019
- WO-A-98/33431
- WO-A-03/032821
- SE-B- 466 379
- US-A- 5 868 668

## Beschreibung

Die Erfindung betrifft ein Spreizlaryngoskop.

Es sind spreizbare Laryngoskope bekannt, welche zwei zueinander parallele Spatel aufweisen, welche einen Arbeitsraum begrenzen, durch welchen Instrumente eingeführt werden können, um einen Eingriff vorzunehmen. Ein derartiges Laryngoskop ist beispielsweise aus DE 199 54 442 bekannt. Dieses Laryngoskop weist zwei Spatel auf, welche mit einem Griffteil verbunden sind. An dem Griffteil ist ein Einstellmechanismus vorgesehen, über welchen die beiden Spatel gespreizt werden können. Dabei werden die Spatel parallel oder winklig verstellt, so dass sie weiter voneinander beabstandet werden, um einen größeren Arbeitsraum zu schaffen. Dabei entstehen seitliche Spalte zwischen den beiden Längsseiten der Spatel. Gemäß DE 199 54 442 A1 sind an einem Spatel bewegliche Klappen angeordnet, um im gespreizten Zustand die seitlichen Spalte zu verschließen und ein Eindringen von Gewebe in die Spalte zu verhindern. Die Anordnung der beweglichen Klappen, hat den Nachteil, dass das Sichtfeld für den Operateur auf das seitlich retraktierte Gewebe stark eingeschränkt wird und zusätzlich durch die schwenkbaren Klappen die Außenabmessungen des Laryngoskops vergrößert werden.

Aus der nachveröffentlichten Druckschrift WO 03/032821 A1 ist ein Untersuchungsinstrument mit zwei auseinander bewegbaren Spateln bekannt, bei welchem einer der Spatel an jeder Längsseite eine Zunge aufweist, welche sich ausgehend vom proximalen Ende des Spatels beabstandet zu dessen Längskante in distaler Richtung erstreckt und bei gespreiztem Instrument den zwischen den Spateln entstehenden Spalt überbrückt. Dadurch wird die seitliche Sicht stark eingeschränkt.

Es ist Aufgabe der Erfindung, ein verbessertes Spreizlaryngoskop zu schaffen, welches kompakter ausgebildet ist und einen besseren Weichteil- und Organschutz insbesondere bei Eingriffen im Bereich des Larynx/Pharynx und ein vergrößertes Sichtfeld für den Operateur ermöglicht.

Diese Aufgabe wird durch ein Spreizlaryngoskop mit den im Anspruch 1 angegebenen Merkmalen gelöst. Bevorzugte Ausführungsformen ergeben sich aus den Unteransprüchen.

Das erfindungsgemäße Spreizlaryngoskop weist wie die bekannten Laryngoskope zwei auseinanderbewegbare Spatel auf. Die beiden Spatel sind vorzugsweise in einem gemeinsamen Griffteil fixiert und können parallel zueinander oder winklig zueinander auseinanderbewegt werden, um einen vergrößerten Arbeitsraum zwischen den Spateln zu schaffen. Erfindungsgemäß sind an jedem der beiden Spatel an jeder seiner Längsseiten jeweils zwei vorstehende Laschen bzw. vorstehende Vorsprünge ausgebildet, welche sich jeweils ausgehend von der jeweiligen Längsseite des einen Spatels in Richtung des anderen Spatels erstrecken. Dabei sind die Laschen starr mit dem jeweiligen Spatel an dessen Längsseiten verbunden. Diese starre Verbindung der Laschen ermöglicht eine kompaktere Ausgestaltung des Laryngoskops als bei Verwendung schwenkbarer Klappen. Ferner wird eine kostengünstigere Herstellung und größere Haltbarkeit erreicht. Die Laschen bzw. Vorsprünge decken Abschnitte des seitlichen Spalts ab, welcher entsteht, wenn die beiden Spatel auseinanderbewegt werden. Dabei überdecken bzw. überbrücken die Laschen in der Querrichtung, d. h. im Wesentlichen normal zu den Spateln, die seitlichen Spalte zwischen den Spateln zumindest teilweise, um ein Eindringen von Gewebe in den Raum zwischen den Spateln zu verhindern. Vorzugsweise erstrecken sich die Laschen nicht über die gesamte Länge der Spatel, sondern nur über kurze Abschnitte in Längsrichtung der Spatel, so dass ein großer Bereich der seitlichen Spalte zwischen den Spateln durch die Laschen nicht verdeckt wird, wodurch ein vergrößertes Sichtfeld für einen Operateur geschaffen wird.

An jedem der beiden Spatel sind an jeder Längsseite der Spatel zwei vorstehende Laschen ausgebildet, welche sich ausgehend von der Längsseite des einen Spatels auf den anderen Spatel zu erstrecken und mit dem Spatel starr verbunden sind. Gemäß dieser Anordnung können auch bei größerem Abstand zwischen den gespreizten Spateln die entstehenden Spalte sicher vor eindringendem Gewebe geschützt werden. Die Anordnung von Vorsprüngen an beiden Spateln ermöglicht dabei bei Verwendung starrer Vorsprünge die Überbrückung bzw. eine Abdeckung auch breiterer Spalte zwischen den Spateln in einer Richtung quer, insbesondere normal zur Längsrichtung der Spatel.

Die an den beiden Spateln ausgebildeten Laschen sind derart gegenüberliegend zueinander angeordnet, dass sich eine Lasche an dem ersten Spatel mit einer Lasche an dem zweiten Spatel zumindest in einem nicht gespreizten Zustand des Laryngoskops überlappen. Bei dieser Anordnung bilden die beiden aneinander gegenüberliegenden Laschen an den beiden Spateln einen gemeinsamen geschlossenen Abschnitt, welcher den seitlichen Spalt bzw. Freiraum zwischen den beiden Spateln überbrückt, um ein Eindringen von Gewebestrukturen und Organteilen zu verhindern. Dabei bilden die beiden einander überlappenden Laschen einen Steg zwischen den beiden Spateln. Durch die überlappende Ausgestaltung kann auch ein großer Spalt bzw. Freiraum zwischen den beiden Spateln überbrückt werden. Da der durch die Laschen gebildete Steg sich in Längsrichtung der Spatel nur über einen schmalen Bereich erstreckt, verbleibt der größte Teil des Spaltes vorzugsweise unverdeckt, wodurch eine gute seitliche Sicht ermöglicht wird.

Bevorzugt erstrecken sich die Laschen im Wesentlichen normal zu den Hauptflächen der Spatel. Die Hauptflächen der Spatel sind jeweils die sich in Längsrichtung erstreckenden Flächen der beiden Spatel, d.h. die eigentlichen Spatelflächen, welche einander gegenüberliegen und die Ober- und Unterseite des Laryngoskops bilden. Das bedeutet, die Laschen erstrecken sich im Wesentlichen parallel zur Bewegungsrichtung der beiden Spatel, wenn diese auseinander bzw. aufeinander zu bewegt werden. Die einander überlappenden Laschen erstrecken sich dabei vorzugsweise parallel zueinander, so dass die Innenseite der einen Lasche an der Außenseite der anderen Lasche anliegt bzw. sich parallel in geringem Abstand zu dieser erstreckt. So wird ein geschlossener Steg geschaffen, welcher ein Eindringen von Weich- bzw. Gewebeteilen verhindern kann. Die Anordnung der Laschen parallel zur Bewegungsrichtung der Spatel ermöglicht einen großen Verstellweg der beiden Spatel zueinander, ohne dass dieser durch die Anordnung der Laschen eingeschränkt wird.

An jeder Längsseite jedes der zwei Spatel sind jeweils zwei Laschen ausgebildet, welche sich im nicht gespreizten Zustand des Laryngoskopes mit zwei gegenüberliegenden Laschen an dem jeweils anderen Spatel überlappen. Dadurch, dass zwei Laschen an jeder Längsseite vorgesehen sind, kann ein Eindringen von Gewebe zuverlässig verhindert werden. Gleichzeitig hat die Anordnung zweier einzelner Laschen gegenüber einer größeren Lasche den Vorteil, dass das Sichtfeld für den Operateur weniger eingeschränkt wird, da die Laschen nur schmale Stege zwischen den Spateln bilden, der übrige Teil der Spalte aber frei bleibt, um eine ausreichende seitliche Sicht zu gewährleisten.

Die zwei Laschen an jeder Seite des Spatels sind vorzugsweise in der Längsrichtung des Spatels voneinander beabstandet. So entsteht ein Freiraum zwischen den beiden Laschen, welcher eine bessere Sicht auf seitlich der Spatel gelegene Gewebeteile während einer Operation zulässt. Gleichzeitig können die voneinander beabstandeten Laschen sicherstellen, dass Weichteile oder Gewebeteile nicht in den Spalt zwischen den beiden Spateln eindringen.

Weiter bevorzugt sind die Laschen so ausgestaltet, dass sie sich auch im gespreizten Zustand des Laryngoskopes mit jeweils einer gegenüberliegenden Lasche an dem jeweils anderen Spatel überlappen. Das bedeutet auch, in jeder gespreizten Position der beiden Spatel überlappen sich die gegenüberliegenden Laschen so, dass ein durchgängig geschlossener Steg zwischen den beiden Spateln gebildet wird, um ein Eindringen von Gewebeteilen in den Spalt zwischen den beiden Spateln zu verhindern. Dabei erstrecken sich die Laschen jedoch in Längsrichtung der Spatel nicht über die gesamte Länge der Spatel, so dass die Sicht auf die seitlich des Laryngoskops gelegenen Gewebeteile möglichst wenig eingeschränkt wird. Das Laryngoskop kann jedoch alternativ auch so ausgestaltet sein oder eingesetzt werden, dass die Spatel weiter voneinander beabstandet bzw. gespreizt werden, so dass die einander gegenüberliegenden Laschen an beiden Spateln sich nicht mehr überlappen und zwischen den Stirnseiten der Laschen ein Spalt entsteht. Dieser Spalt ist jedoch stets wesentlich schmaler als der übrige entstehende Spalt zwischen den beiden Spatel, so dass auch bei einer solchen Anordnung ein Eindringen von Gewebestrukturen in den jeweiligen seitlichen Spalt zwischen den beiden Spateln verhindert werden kann. Gleichzeitig wird die seitliche Sicht so wenig wie möglich eingeschränkt.

Vorzugsweise sind die Laschen einstückig mit dem jeweils zugehörigen Spatel ausgebildet. Beispielsweise können die Laschen gemeinsam mit dem zugehörigen Spatel aus einem Metallblech durch entsprechendes Zuschneiden und Umformen hergestellt werden. Dies ermöglicht eine kostengünstige Fertigung des Laryngoskops.

Nachfolgend wir die Erfindung beispielhaft anhand der beigefügten Figuren beschrieben. In diesen zeigt:
Figur 1 eine geschnittene Seitenansicht des erfindungsgemäßen Laryngoskops im nicht gespreizten Zustand und
Figur 2 eine geschnittene Seitenansicht des erfindungsgemäßen Laryngoskops im gespreizten Zustand.

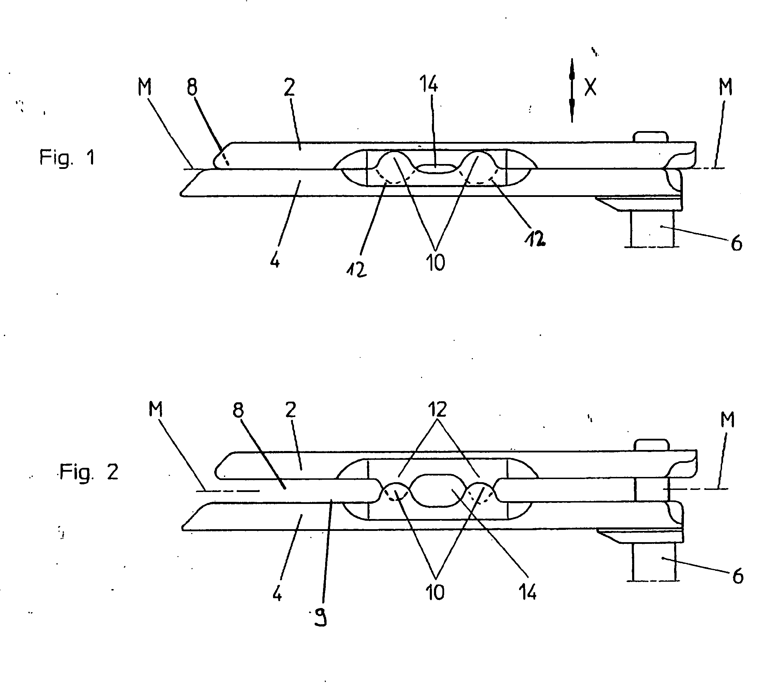

Das erfindungsgemäße Laryngoskop gemäß einer bevorzugten Ausführungsform wird zunächst im nicht gespreizten Zustand anhand von Figur 1 erläutert. Das Laryngoskop weist wie bekannte Spreizlaryngoskope zwei sich parallel zueinander erstreckende Spatel 2 und 4 auf. Die Spatel 2 und 4 sind in einem Griffbereich 6 derart miteinander verbunden, dass sie in der Richtung X auseinanderbewegt werden können. Dabei werden die Spatel 2 und 4 entweder so bewegt, dass sie sich ständig parallel zueinander erstrecken oder sie werden um Drehachsen im Griffbereich 6 auseinandergeschwenkt, so dass die distalen Enden der Spatel 2 und 4, d. h. die dem Griffbereich 6 entgegengesetzten Enden, in der Richtung X weiter voneinander beabstandet werden. Die beiden Spatel 2 und 4 weisen jeweils einen im Wesentlichen U-förmigen Querschnitt auf, und umschließen gemeinsam einen Arbeitsraum bzw. Arbeitskanal 8, durch welchen Hilfsinstrumente für eine Operation eingeführt werden können. Wenn die beiden Spatel 2 und 4 in der Richtung X auseinanderbewegt werden, wird der Arbeitskanal 8 entsprechend vergrößert, um einen größeren Arbeitsraum für eine Operation zu schaffen.

Erfindungsgemäß sind an jeder Längsseite jedes Spatels 2, 4 jeweils zwei Laschen 10, 12 ausgebildet. Dabei erstrecken sich die Laschen 10 ausgehend von dem Spatel 4 über die Mittelebene M hinaus in Richtung des Spatels 2. Die Laschen 12 erstrecken sich ausgehend vom dem Spatel 2 über die Mittelebene M hinaus in Richtung des Spatels 4. Die Laschen 10 und die Laschen 12 (gestrichelt dargestellt) sind jeweils in Längsrichtung des Spatels 2, 4, d. h. in einer Richtung parallel zur Mittelebene M und normal zur Richtung X, voneinander beabstandet, so dass zwischen den Vorsprüngen bzw. Laschen 10, 12 ein Freiraum 14 verbleibt, welcher die Sicht auf seitlich gelegene Gewebeteile verbessert. Die Laschen sind in Längsrichtung der Spatel im Wesentlichen im Mittelbereich der Spatel angeordnet, so dass auch distalwärts und proximalwärts der Laschen die Spalte nicht verdeckt werden und eine gute Sicht auf seitliche Gewebeteile zulassen.

Die Laschen 10 und 12 erstrecken sich in parallel zueinander gelegenen Ebenen und überlappen einander. Dabei erstrecken sich die Laschen, 10, 12 vorzugsweise im Wesentlichen parallel zur Richtung X, so dass die Laschen 10 an den Außenseiten der Laschen 12 anliegen bzw. sich in geringem Abstand parallel zu diesen erstrecken, wenn die beiden Spatel 2 und 4 auseinanderbewegt werden (siehe Figur 2). Die Laschen 10 und 12 weisen in der Richtung X bevorzugt jeweils derartige Längen auf, dass auch in dem Zustand, in dem die Spatel 2 und 4 am weitesten auseinanderbewegt sind, der zwischen den Spateln 2 und 4 gebildete Spalt 9 durch die Laschen 10 und 12 in der Richtung X vollständig überdeckt bzw. überbrückt wird. Auf diese Weise werden durch die Laschen 10 und 12 zwei durchgängig geschlossene Stege zwischen den Spateln 2 und 4 gebildet, auch wenn diese sich in der am weitesten voneinander beabstandeten Position befinden, so dass ein Eindringen von Weichteilen oder anderen Gewebeteilen durch die seitlichen Spalte in den Arbeitsraum 8 verhindert werden kann. Die Laschen 10, 12 sind jeweils vorzugsweise einstückig mit den Seitenwandungen des Spatels 2 und 4 ausgebildet. Sie bilden jeweils in Richtung des jeweils anderen Spatels vorstehende Verlängerungen der Seitenteile der Spatel 2 und 4. So können die Spatel 2 und 4 gemeinsam mit den jeweiligen Vorsprüngen 10 und 12 leicht einstückig aus einem Blechzuschnitt durch Umformen gefertigt werden. Ferner wird eine insgesamt kompaktere Ausgestaltung des Laryngoskops möglich.

Figur 2 zeigt das Laryngoskop gemäß Figur 1 im gespreizten Zustand, in dem die beiden Spatel 2 und 4 weiter voneinander beabstandet liegen, so dass die entlang der beiden Längsseiten des Laryngoskops erstreckenden Spalte 9 entstehen bzw. sich vergrößern. Auch in diesem gespreizten Zustand überlappen sich die Laschen 10 und 12 noch im Bereich ihrer äußeren Enden, so dass die Spalte in einer Richtung normal zur Längsachse bzw. zur Mittelebene M vollständig überbrückt werden, um ein Eindringen von Gewebeteilen zu verhindern. Seitlich und zwischen den Laschen 10 und 12 bleibt der Spalt 9 frei bzw. verbleibt der Freiraum 14, so dass eine gute seitliche Sicht gewährleistet beleibt. Wenn die beiden Spatel 2 und 4 weiter gespreizt werden, werden sich die Laschen 10 und 12 ab einer bestimmten Spreizung nicht mehr überlappen, dennoch überbrücken bzw. überdecken sie in Querrichtung weiterhin einen großen Bereich der Spalte 9, so dass weiterhin ein Eindringen von Gewebeteilen in die Spalte verhindert werden kann.

Die Vorsprünge 10 und 12 können auch bei einem großen Abstand der Spatel 2 und 4 zueinander die seitliche Spalte so überbrücken, dass kein Gewebe in den Zwischenraum zwischen den Spateln 2 und 4 eindringen kann. Gleichzeitig überdecken die Vorsprünge bzw. Laschen 10 und 12 jedoch nur sehr kleine seitliche Bereiche des zwischen den Spateln 2 und 4 entstehenden Spaltes 9, so dass die seitliche Sicht kaum eingeschränkt wird. Die Laschen 10, 12, die hier nur am Beispiel eines Spaltes 9 beschrieben wurden, sind an dem gegenüberliegenden Längsspalt 9 zwischen den beiden Spatel 2, 4 entsprechend ausgebildet, so dass der Arbeitskanal 8 von beiden Längsseiten gegen Eindringen von Gewebe geschützt ist.

### Bezugszeichenliste

- 2, 4: Spatel
- 6: Griffbereich
- 8: Arbeitskanal
- 9: Spalte
- 10, 12: Vorsprünge
- 14: Freiraum
- M: Mittelebene
- X: Bewegungsrichtung

## Patentansprüche

1. Spreizlaryngoskop mit zwei auseinanderbewegbaren Spateln (2, 4), wobei an jeder Längsseite jedes der beiden Spatel (2, 4) jeweils zwei vorstehende Laschen (10, 12) ausgebildet sind, welche starr mit den Spatel (2, 4) verbunden sind, sich ausgehend von der Längsseite des einen Spatels (2, 4) auf den anderen Spatel (2, 4) zu erstrecken und welche sich im nicht gespreizten Zustand des Laryngoskopes mit zwei gegenüberliegenden Laschen (10, 12) an dem jeweils anderen Spatel (2, 4) überlappen.

2. Spreizlaryngoskop nach Anspruch 1, bei welchem sich die Laschen (10, 12) im Wesentlichen normal zu den Hauptflächen der Spatel (2, 4) erstrecken.

3. Spreizlaryngoskop nach einen der vorangehenden Ansprüche, bei welchem die zwei Laschen (10, 12) an jeder Seite des Spatels (2, 4) in der Längsrichtung des Spatels (2, 4) voneinander beabstandet sind.

4. Spreizlaryngoskop nach einem der vorangehenden Ansprüche, bei welchem die Laschen (10, 12) so ausgestaltet sind, dass sie sich im gespreizten Zustand des Laryngoskopes mit jeweils einer gegenüberliegenden Lasche (10, 12) an dem jeweils anderen Spatel (2, 4) überlappen.

5. Spreizlaryngoskop nach einem der vorangehenden Ansprüche, bei welchem die Laschen (10, 12) einstückig mit dem jeweils zugehörigen Spatel (2, 4) ausgebildet sind.

## Claims

1. A spreading laryngoscope with two spatulas (2,4) able to be moved apart, wherein in each case two projecting tabs (10, 12) are formed on each longitudinal side of the two spatulas (2, 4), wherein the tabs are rigidly connected to the spatula (2, 4), proceeding from the longitudinal side of the one spatula (2,4), extend to the other spatula (2, 4), and in the non-spread condition of the larynogoscope overlap with two oppositely lying tabs (10, 12) on the respective other spatula (2, 4).

2. A spreading laryngoscope according to claim 1, with which the tabs (10, 12) extend essentially normally to the main surfaces of the spatulas (2, 4).

3. A spreading laryngoscope according to one of the preceding claims, with which the two tabs (10, 12) on each side of the spatula (2, 4) are distanced from one another in the longitudinal direction of the spatula (2, 4).

4. A spreading laryngoscope according to one of the preceding claims, with which the tabs (10, 12) are designed such that in the spread condition of the laryngoscope they overlap in each case with one oppositely lying tab (10, 12), on the respective other spatula (2, 4).

5. A spreading laryngoscope according to one of the preceding claims, with which the tabs (10, 12) are formed as one piece with the respective associated spatula (2, 4).

## Revendications

1. Laryngoscope à section écartable comprenant deux spatules (2, 4) pouvant être écartées l'une de l'autre, dans lequel deux languettes (10, 12) en saillie sont réalisées sur chaque côté longitudinal de chacune des deux spatules (2, 4), lesquelles languettes sont reliées rigidement aux spatules (2, 4), s'étendent à partir du côté longitudinal d'une spatule (2, 4) vers l'autre spatule (2, 4) et se chevauchent avec deux languettes (10, 12) en vis à vis sur l'autre spatule (2, 4) lorsque le laryngoscope n'est pas en position écartée.

2. Laryngoscope à section écartable selon la revendication 1, dans lequel les languettes (10, 12) s'étendent sensiblement perpendiculairement aux surfaces principales respectives des spatules (2, 4).

3. Laryngoscope à section écartable selon l'une des revendications précédentes, dans lequel les deux languettes (10, 12) sont espacées l'une de l'autre de chaque côté de la spatule (2, 4) dans la direction longitudinale de la spatule (2, 4).

4. Laryngoscope à section écartable selon l'une des revendications précédentes, dans lequel les languettes (10, 12) sont aménagées de telle sorte qu'elles se chevauchent avec la languette respective (10, 12) en vis à vis sur l'autre spatule (2, 4) lorsque le laryngoscope est en position écartée.

5. Laryngoscope à section écartable selon l'une des revendications précédentes, dans lequel les languettes (10, 12) sont réalisées d'une seule pièce avec la spatule (2, 4) concernée.
